# EUROPEAN PATENT SPECIFICATION

(11) **EP 3 717 657 B1**
(45) Date of publication and mention of the grant of the patent: **05.08.2026**
(21) Application number: 18816284.6
(22) Date of filing: 30.11.2018
(51) Int. Cl.: C12Q 1/04, G01N 35/00, G01N 33/569, C12Q 1/18, B01L 3/00, G01N 35/02

(54) **A SYSTEM FOR ASSESSING MICROBIAL SUSCEPTIBILITY TO ANTIBIOTIC AGENTS**
SYSTEM ZUR BEWERTUNG DER MIKROBIELLEN ANFÄLLIGKEIT GEGENÜBER ANTIBIOTIKA
SYSTÈME D'ÉVALUATION DE LA SENSIBILITÉ MICROBIENNE À DES AGENTS ANTIBIOTIQUES

(30) Priority: 30.11.2017 SE 1751473
(43) Date of publication of application: 07.10.2020
(73) Proprietor: Ascilion AB, 164 25 Kista (SE)
(72) Inventor: RANGSTEN, Pelle, 743 40 Storvreta (SE); RENLUND, Markus, 184 60 Åkersberga (SE); FRANZÉN, Mikael, 178 51 Ekerö (SE); HILLMERING, Mikael, 192 73 Sollentuna (SE)
(74) Representative: Zacco Sweden AB
(86) International application number: PCT/SE2018/051236
(87) International publication number: WO 2019/108124

(56) References cited:
- WO-A1-2010/048511
- WO-A1-2013/130875
- WO-A1-2016/150871
- WO-A1-2016/207065
- WO-A1-2017/182645
- WO-A1-2017/185012
- WO-A2-2016/161022
- US-A- 4 132 599
- US-A1- 2003 138 874
- US-A1- 2014 335 556
- US-A1- 2016 349 178
- US-A1- 2017 298 408
- SHARMA SANJIV ET AL: "Evaluation of a minimally invasive glucose biosensor for continuous tissue monitoring", ANALYTICAL AND BIOANALYTICAL CHEMISTRY, SPRINGER BERLIN HEIDELBERG, DE, vol. 408, no. 29, 15 October 2016 (2016-10-15), pages 8427 - 8435, XP036103245, ISSN: 1618-2642, [retrieved on 20161015], DOI: 10.1007/S00216-016-9961-6
- HYUNJAE LEE ET AL: "Wearable/disposable sweat-based glucose monitoring device with multistage transdermal drug delivery module", SCIENCE ADVANCES, 8 March 2017 (2017-03-08), pages e1601314, XP055511563, Retrieved from the Internet <URL:http://advances.sciencemag.org/content/advances/3/3/e1601314.full.pdf> [retrieved on 20181002], DOI: 10.1126/sciadv.1601314
- OLESZKIEWICZ A ET AL: "The confounding effect of background odors on olfactory sensitivity testing", JOURNAL OF NEUROSCIENCE METHODS, 3 May 2007 (2007-05-03), Netherlands, pages 366 - 371, XP055817916, Retrieved from the Internet <URL:https://www.ncbi.nlm.nih.gov/pmc/articles/PMC2769590/pdf/dst-01-0366.pdf> [retrieved on 20210624], DOI: 10.1016/j.jneumeth.2018.05.012
- GES I A ET AL: "Enzyme electrodes to monitor glucose consumption of single cardiac myocytes in sub-nanoliter volumes", BIOSENSORS AND BIOELECTRONICS, ELSEVIER SCIENCE LTD, UK, AMSTERDAM , NL, vol. 25, no. 5, 15 January 2010 (2010-01-15), pages 1019 - 1024, XP026808891, ISSN: 0956-5663, [retrieved on 20090919]
- ROSARIO ESPOSITO ET AL: "Glucose Sensing by Time-Resolved Fluorescence of Sol-Gel Immobilized Glucose Oxidase", SENSORS, vol. 11, no. 4, 24 March 2011 (2011-03-24), pages 3483 - 3497, XP055547722, DOI: 10.3390/s110403483
- REN ZHONG ET AL: "Application of time-resolved glucose concentration photoacoustic signals based on an improved wavelet denoising", VISUAL COMMUNICATIONS AND IMAGE PROCESSING; 20-1-2004 - 20-1-2004; SAN JOSE,, vol. 9273, 29 October 2014 (2014-10-29), pages 92733G - 92733G, XP060043416, ISBN: 978-1-62841-730-2, DOI: 10.1117/12.2070843
- ÖZDEN BALTEKIN ET AL: "Antibiotic susceptibility testing in less than 30 min using direct single-cell imaging", PROCEEDINGS OF THE NATIONAL ACADEMY OF SCIENCES OF THE UNITED STATES OF AMERICA, vol. 114, no. 34, 8 August 2017 (2017-08-08), US, pages 9170 - 9175, XP055548101, ISSN: 0027-8424, DOI: 10.1073/pnas.1708558114
- KIM SEUNGWAN ET AL: "In vitro monitoring of goat-blood glycemia with a microwave biosensor", CURRENT APPLIED PHYSICS, vol. 14, no. 4, 31 January 2014 (2014-01-31), pages 563 - 569, XP028841468, ISSN: 1567-1739, DOI: 10.1016/J.CAP.2014.01.011

## Description

### TECHNICAL FIELD

The present invention generally relates to the field of microbial measurements, and more specifically to the field of systems and devices for assesseing susceptibility to agents with antibiotic activity.

### BACKGROUND ART

A common problem in clinical settings is to measure the efficiency of an antibiotic on a sample with bacteria. This is conventionally performed by providing a sample with bacteria to an Agar plate with different disks containing antibiotic compounds. The agar plate is then placed in an environment suitable for microbial growth. After an incubation time of approximately 24 hours the agar plate is then analysed. If the antibiotic is efficient a ring of dead bacteria is formed around each disk with antibiotic. The diameter of the ring is proportional to the efficiency of the antibiotic.

It is known in the art (e.g. JP57068796, WO2016/150871) to use pH indicators to detect the acidic degradation products of glucose metabolism in *Enterobacteriaceae* when cultured in the presence or absence of antibiotics, in order to assess antibiotic susceptibility.

WO2015/107054 discloses a mass spectrometric method for determining microbial resistance to antibiotics. The method is based on growing microbes in a culture medium and mass spectrometric measurement of a nutrient component, such as a peptide, or a chemically modified variant of the nutrient component, wherein a decrease in the nutrient component or an increase in the chemically modified variant of the nutrient component indicates resistance to the antibiotic. This method requires removal of culture medium for measurement in a separate mass spectrometer. The method also requires a fully synthetic culture medium, which has clean mass spectra without much chemical background noise.

Sine et al. have suggested prediction of antibiotic resistance through measurement of glucose consumption (Sine et al., poster 099, 111th General Meeting American Society for Microbiology, 2011).

Measurement of glucose concentrations using microwaves is known through i.a. Kim Seungwan et al, "In vitro monitoring of goat-blood glycemia with a microwave biosensor", Current Applied Physics, (20140131), vol. 14, no. 4, pages 563 - 569 Gianluca Gennarelli et al, "A Microwave Resonant Sensor for Concentration Measurements of Liquid Solutions", leee Sensors Journal (201305), vol. 13, no. 5,Yun Fan et al., "Testing glucose concentration in aqueous solution based on microwave cavity perturbation technique", Biomedical Engineering and Informatics (BMEI), 2010.

WO2013130875 describes methods, compositions, kits and systems for rapid determination of antibiotic susceptibility of a microbe, wherein the microbe is extracted or concentrated from a test sample using a microbe- targeting substrate, wherein the microbe-targeting substrate comprises on its surface a microbe-binding molecule.

WO2017185012 describes automated rapid antimicrobial susceptibility testing systems.

US2016349178 describes a device and method for determining antimicrobial resistance status of a microorganism from a positive sample bottle, based on detection of light transmitted or scattered by the sample.

US4132599 describes a method for the quick determination of the susceptibilities of various unidentified bacteria contained in an aqueous physiological fluid sample, particularly urine, to one or more antibiotics, based on a pre-screen of the presence of bacterial ATP in an assay and subsequent exposure of the bacteria to one or more antibiotics to assess growth.WO2016161022 describes a system for automated microorganism identification and antibiotic susceptibility testing comprising an optical detection system configured to obtain darkfield and fluorescence photomicrographs of one or more microorganisms contained in a plurality of microfluidic sample channels and a controller configured to process microorganism information derived from photomicrographs obtained by the optical detection system.

### SUMMARY OF THE INVENTION

There exists a need for systems capable of providing for rapid assessments of whether a microorganism is susceptible to an antibiotic. Such systems preferably provide the user with information on what type of antibiotic may be used and the concentrations that are needed to inhibit the growth of, or kill, the microorganism.

The present invention aims to provide such systems.

The present invention builds on the understanding that if a sample contains microorganisms and glucose is added to the sample, the glucose concentration will decrease over time due to the glucose consumption by the microorganisms in the sample. When the microorganisms are cultivated in the presence of an antibiotic agent, the glucose concentration will be constant or only slowly decrease over time if the microorgansims are susceptible to the antibiotic activity. If the microorganisms are resistant to the antibiotic activity, the glucose concentration will decrease at the same rate as when no antibiotic agent is present. It is consequently possible to directly assess the susceptibility of the microorganism to the antibiotic agent by measuring the rate of decrease in glucose in the presence and absence of the antibiotic agent and comparing the respective rates.

Thus, in a first aspect, the present invention relates to a system for analysing susceptibility of a glucose metabolizing microorganism to an antibiotic agent, configured for holding at least a first and a second culture medium containers configured for holding
i. a volume of culture medium containing glucose;
ii. a sample containing a glucose metabolizing microorganism; and
iii. a predetermined concentration of the antibiotic agent;
and further configured to support culture of said glucose metabolizing microorganism for a period of time;
said system comprising
- a glucose sensor capable of measuring the glucose concentration in said culture medium in said first and second culture medium containers to provide time-resolved glucose concentration data during said period of time; and
- a computer configured to retrieve for each culture medium container:
   i. data comprising the amount and/or concentration of the antibiotic agent; and
   ii. the time-resolved glucose concentration data;
   said computer being programmed to calculate a susceptibility score reflecting the susceptibility of the glucose metabolizing microorganism to an antibiotic activity of the antibiotic agent based on a relation between the predetermined concentration of the antibiotic agent and the time-resolved glucose concentration data.

In one embodiment, the system further comprises an agitation plate configured for holding and agitating at least one blood culture bottle.

In one embodiment, the system further comprises a robotic arm provided with a pipetting unit for transferring a predetermined amount of culture medium from a blood culture bottle to at least one culture medium container.

In one embodiment, the system further comprises a housing covering at least said culture medium containers and said blood culture bottles, said housing being configured to maintain a controlled temperature within said housing.

In one embodiment, said housing is provided with a loading dock for loading blood culture bottles into said system.

In one embodiment, the system further comprises a robotic arm provided with a grip for moving a blood culture bottle from the loading dock to the agitation plate.

In one embodiment,the pipetting unit and the grip described above are provided on a single robotic arm.

In one embodiment, the glucose sensor comprises an antenna configured to excite an electromagnetic wave in a measurement chamber and a sample holder configured to hold a culture medium sample in a position in the measurement chamber. The measurement chamber may be dimensioned to be in resonance for a predetermined frequency of the electromagnetic wave. The sample holder may be a through hole for a capillary extending through the measurement chamber.

In one embodiment, the glucose sensor comprises an enzyme electrode.

In one embodiment, the system is configured for holding a plurality of culture medium containers arranged in an array of columns and rows. Such an array may consist e.g. of 6 (2*3), 8 (2*4) 12 (3*4) 24 (4*6); 48 (6*8); 96 (8*12); or 384 (16*24) culture medium containers.

In one embodiment, the system comprises a number of glucose sensors equal to the number of columns or rows in the array.

In one embodiment, the system comprises a number of culture medium containers equals the number of glucose sensors.

In one embodiment, the system is configured for holding culture medium containers having a volume in the range of 0.1-2 mL.

In one embodiment, the system comprises means for automatically dispensing a predetermined amount of culture medium in culture medium containers.

In one embodiment, the system comprises means for controlling the temperature of the culture medium containers.

In one embodiment, the system comprises means for shaking or agitation of the culture medium containers.

### BRIEF DESCRIPTION OF THE DRAWINGS

Embodiments of the invention will now be explained in detail, by way of example only, with reference to the accompanying figures, in which:
Figure 1 is an illustration of the core components of the system.
Figure 2 is an illustration of one embodiment of the system.
Figure 3 is an illustration of a further embodiment of the system.
Figure 4 is an illustration of a glucose sensors using an electromagnetic field to measure the glucose concentration.
Figure 5 is an illustration of glucose sensors useful in the system. (A) Cross-section of an individual sensor; (B) Perspective view of an individual sensor; (C) Cross-section of array of eight sensors; (D) Array of eight sensors.
Figure 6 is a flow chart of a method that may be implemented using the system according to the present invention.
Figure 7 is a flow chart of a method that may be implemented using the system according to the present invention.
Figure 8 is a flow chart of a method that may be implemented using the system according to the present invention.
Figure 9 is a diagram showing time-resolved glucose concentration data obtained for *E.coli*
Figure 10 is a diagram showing time-resolved glucose concentration data obtained for *E.faecalis*
Figure 11 is a diagram showing time-resolved glucose concentration data obtained for *S.aureus.*

### DEFINITIONS

All terms used herein are intended to have the meaning given to them by the person skilled in the art. For the sake of clarity, a few terms are further defined below.

The terms "antibiotic agent" or "agent with antibiotic activity" shall be considered equivalent and relate to agents that suppress or inibit the growth or viability of microorganisms, such as bacteria, archaebacteria, protozoa and yeasts. Such agents include chemical compounds, ultraviolet light, radiation, heating, microwaves etc.

### DETAILED DESCRIPTION

In a first aspect, the present invention relates to a system for analysing susceptibility of a glucose metabolizing microorganism to an antibiotic agent, configured for holding at least a first and a second culture medium containers configured for holding
iii. a volume of culture medium containing glucose;
iv. a sample containing a glucose metabolizing microorganism; and
v. a predetermined concentration of the antibiotic agent;
and further configured to support culture of said glucose metabolizing microorganism for a period of time;
said system comprising
- a glucose sensor capable of measuring the glucose concentration in said culture medium in said first and second culture medium containers to provide time-resolved glucose concentration data during said period of time; and
- a computer configured to retrieve for each culture medium container:
   i. data comprising the amount and/or concentration of the antibiotic agent; and
   ii. the time-resolved glucose concentration data;
   said computer being programmed to calculate a susceptibility score reflecting the susceptibility of the glucose metabolizing microorganism to an antibiotic activity of the antibiotic agent based on a relation between the predetermined concentration of the antibiotic agent and the time-resolved glucose concentration data.

The culture medium containers may be arranged in or on a separate device, such as a microtitre plate as further described below, which is introduced into the system on use. The system is consequently configured for holding these culture medium containers. It is also possible configure the system so that the culture medium containers are permanently mounted in the system.

The system may also comprise a rack for holding a plurality of blood culture bottles. Blood culture bottles are routinely used for culturing patient blood samples suspected of containing microorganisms, in particular pathogenic microorganisms. Providing a rack for holding blood culture bottles within the system keeps the blood culture bottles in proximity to the culture medium containers and facilitates easy and frequent transfer of aliquots from the blood culture bottles to the culture medium containers for subsequent analysis for antimicrobial susceptibility. Antimicrobial susceptibility can only be determined when a sufficient number of viable microorganisms are present in the culture medium containers. It has therefore been the practice to culture blood samples in blood culture bottles for a standard time that ensures that there is a sufficient number of viable microorganisms, or until the blood culture bottle is indicated as positive. Frequent sampling of the blood culture bottles, made possible by keeping them within the system, in combination with the glucose concentration based susceptibility testing, makes it possible to asses the sampled microorganisms' susceptibility to antibiotic agents as soon as there are a sufficient concentration of viable microorganisms in the blood culture bottle. This significantly reduces the time required from obtaining the sample from the patient to the assessment of what antibiotic agent may be used to combat the infection.

The rack for holding the plurality of blood culture bottles is preferably arranged to agitate the blood culture bottles. Agitation provides optimal culture conditions to any microorganisms present in the patient sample.

It is also preferable to provide the system with a robotic arm provided with a pipetting unit configured for transferring a predetermined amount of blood culture medium from a blood culture bottle to at least one culture medium container.

The system may further be provided with a culture medium storage container for storing fresh culture medium. A pipetting unit provided on a robotic arm may then be configured for transferring a predetermined amount of fresh culture medium to the culture medium containers as needed. This pipetting unit and robotic arm may be the same as the pipetting unit and robotic arm described above.

In one embodiment, the system further comprises a housing covering at least said culture medium containers and said blood culture bottles, said housing being configured to maintain a controlled temperature within said housing. In particular, the housing may be configured to maintain a temperature, such as 37°C, to provide optimal growth conditions for any microorganisms present in the blood culture bottles or culture medium containers.

In one embodiment, the housing is provided with a loading dock for loading blood culture bottles into said system. This facilitates that a user places a blood culture bottle in the loading dock, whereafter the blood culture bottle is transported by the system to the holding rack for blood culture bottles described above. The transportation may be effected e.g. by a robotic arm. The loading dock may typically comprise an outer door between the outside of the housing and the interior of the loading dock, and an inner door between the interior of the loading dock and the interior of the housing.

The system may also be equipped with a barcode reader configured to read a barcode on a blood culture bottle as it is placed in the loading dock or transported to the holding rack. This makes it possible to identify and register each individual bottle loaded into the system and register its position on the holding rack. Samples of culture medium can then be transported from an individual blood culture bottle to one or more culture medium containers and information relating to the identity of the blood culture bottle and the identity and location of the culture medium containers is registered by the system. The culture medium containers are then stored under conditions suitable for microbial growth and analysed for decreased glucose concentration as described, whereafter the results of the analysis can be attributed to the microbial content in the respective blood culture bottle.

The glucose sensor is preferably movably mounted on a robotic arm or the like, so that it can be moved to the positions of the culture medium containers in order to perform glucose concentration measurements. Alternatively it is mounted in a fixed position in the system and a liquid handling system is configured to provide sample aliquots of the culture media to the sensor. The glucose sensor could be provided to measure glucose concentration momentarily or continuously. A plurality of sensors could be provided to measure the glucose concentration in a plurality of culture medium containers simultaneously.

The system preferably further comprises a liquid handling robot that is configured to transfer liquids within the system. Such liquid handling robots are well-known in the art and commercially available from e.g. Tecan Group Ltd., Switzerland, but can also be designed and manufactured specifically for implementing the invention.

On a general level, such liquid handling robots comprise a workbench where modules performing various functions are mounted and one or more robotic arms that can move liquids, plates etc. around the workbench. A pipetting unit is usually included mounted on a robotic arm and configured to obtain disposable pipette tips from a pipette tip rack, obtain a specified amount of liquid from one container and transferring it to another, and then dispose the pipette tip in a waste container.

In one embodiment, the system comprises means for automatically dispensing a predetermined amount of culture medium in culture medium containers. Such means may include a culture medium storage container and a liquid handling robot configured to transfer culture medium from said culture medium storage container to the culture medium containers adapted for cultivating microorganisms. The culture medium storage containers may be blood culture bottles or a culture medium storage container for storing fresh culture medium as described above.

The culture medium containers may be of any type, size and material suitable for cultivation of microorganisms. In a presently preferred embodiment the culture medium containers have a volume in the range 0.1 to 2.0 mL. The containers may be arranged in an array of columns and rows, such as arranged as wells in a microtitre plate. Such an array may be in a 6 (2*3), 8 (2*4) 12 (3*4) 24 (4*6); 48 (6*8); 96 (8*12); 384 (16*24) format, preferably a 96 well format. Microtitre plates for cell cultivation are well known in the art and commercially available from a wide range of suppliers (e.g. Greiner Bio-One, Thermo Fisher Scientific).

When the culture medium containers are arranged in or on a separate device, it is preferable to provide devices comprising a plurality of culture medium containers each having a predetermined amount of a pre-determined antibiotic agent deposited therein. When a predetermined volume of culture medium is added to the culture medium containers, the predetermined concentration of antibiotic agent is achieved. Microtitre plates prepared with increasing amounts of antibiotic agents in series of wells are known in the art for veterinary applications, e.g. from Statens Veterinärmedicinska Anstalt, SVA, Uppsala, Sweden, under the trade name VetMIC.

In one embodiment, the system comprises a number of glucose sensors equal to the number of columns or rows in the array

Predetermined amounts of antibiotic agents may be deposited in the culture medium containers before culture medium is added. The amount of antibiotic agent is then determined as the amount necessary to obtain the desired predetermined concentration in the culture medium once the culture medium is added to the container.

The system may also comprise containers configured for holding stock solutions of antibiotic agents. In this embodiment, a liquid handling robot is configured to obtain appropriate volumes of the stock solutions for transfer to the relevant culture medium containers so as to achieve the predetermined concentration in the culture medium in the respective container.

The system may further comprise a mount for mounting the culture medium containers. Said mount may be configured to provide shaking or agitation to the culture medium containers in order to facilitate and/or promote the growth of the microorganisms in the containers. Such shakers are also well-known in the art and available from i.a. commercial suppliers such as Eppendorf, Thermo Fisher Scientific etc.

The system may further comprise a module configured to control the temperature in or around the culture medium containers, such as within the housing described above, in order to facilitate and/or promote the growth of the microorganisms in the culture medium containers and/or blood culture bottles. Such module preferably comprise a temperature sensor to measure the temperature in the system, of the air around the culture medium containers and/or blood culture bottles, or of the culture medium itself. The temperature sensor may be connected to heating and cooling means to provide heating and/or cooling as necessary to facilitate and/or promote the growth of the microorganisms in the containers. Heating and/or cooling may be provided i.a. through the mount in which the cell culture medium containers are mounted during use of the system, e.g. by the use of a heating table, or through circulation of heated or cooled air or gas around the containers, or within the housing, during use of the system.

In a further aspect, the invention relates to an array of glucose sensors, said array being configured for use in a system according to the invention. This aspect also includes the use of such an array in a system according to the invention.

In a further aspect, the invention relates to an array of glucose sensors comprising a plurality of glucose sensors arranged along a straight line. Such arrays are useful in connection with the system according to the invention.

In one embodiment, each glucose sensor in the array comprises an area that in use is in contact with the culture medium, wherein said array is arranged so that the centres of said areas are spaced 4-30 mm, such as 9 mm or 4.5 mm, apart. This makes the array useful in connection with microtitre plates as culture medium containers. The distance between the areas is measured from center to center. The area in contact with the culture medium may be an area where the glucose sensor interacts with glucose molecules in the medium in order to detect the glucose molecules, or it may be a tip of a tube arranged to withdraw a small portion of the culture medium in case the detection of glucose molecules is performed outside the container.

In one embodiment, each glucose sensor in the array comprise an enzyme electrode or a measurement chamber dimensioned to be in resonance for a predetermined frequency of an electromagnetic wave.

The system according to the invention will now be described with reference to the appended drawings.

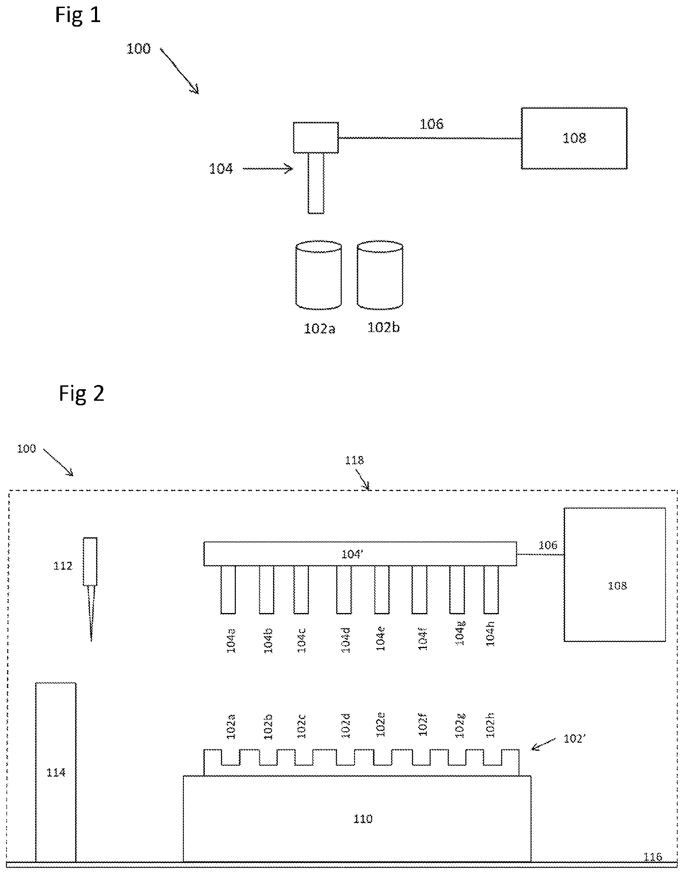

Figure 1 is an illustration of the core components of the system (100) when in use. At least a first (102a) and a second (102b) culture medium containers are included or inserted into the system. The culture medium containers are configured to hold a volume of culture medium containing glucose, a sample containing a glucose metabolizing microorganism; and a predetermined concentration of the antibiotic agent. The system further includes a glucose sensor (104) capable of measuring the glucose concentration in said culture medium in said first and second culture medium containers to provide time-resolved glucose concentration data during said period of time. The glucose sensor (104) is connected to a communication line (106) configured to convey the time-resolved glucose concentration data from the glucose sensor (104) to a computer (108) being programmed to calculate a susceptibility score reflecting the susceptibility of the glucose metabolizing microorganism to an antibiotic activity of the antibiotic agent based on a relation between the predetermined concentration of the antibiotic agent and the time-resolved glucose concentration data obtained from said first and second culture medium containers (102a, 102b).

The communication line (106) may be a wired or a wireless communication line.

Data on the predetermined concentration of the antibiotic agent for each culture medium container may be provided to the computer either by a user or through scanning a predefined code on the culture medium containers, such as a bar code or QR-code.

Figure 2 is an illustration of one embodiment of the system according to the invention. An array (102') of culture medium containers (102a-102h) is arranged on a mount (110) providing agitation, shaking and/or heat to the containers in order to promote growth of microorganisms therein. In the figure, a single column of culture medium containers 102 are shown, but additional columns may be incorporated. Typically, in a 96-container format, 12 columns of containers are included.

An array (104') of glucose sensors (104a-104h) is mounted on a robotic arm (not shown) configured to move the array (104') of sensors into a position where sensors are in contact with the culture media in the containers (102a-102h), and, in turn, the additional columns of containers in the array (102') of containers. The array (104') of sensors may be moved to a wash station (not shown) between measurements if necessary. If the parts of the glucose sensors (104) in contact with the culture medium are disposable, the array (104') of sensors is preferably moved to a waste container (not shown) where the disposable parts are discarded and the moved to a rack comprising new parts for mounting on the array (104') of glucose sensors. Alternatively, the entire array (104') is made disposable and discarded between measurements.

As in Figure 1, the array (104') of glucose sensors is connected to the computer (108) through a communication line (106).

The system further comprise a pipetting head (112) for transferring culture medium from a culture medium storage container (114) to the culture medium containers (102a-102h).

All modules are mounted on a workbench (116). The components of the system are contained in a housing (118), shown in dashed line, mounted on the edges of the workbench (116).

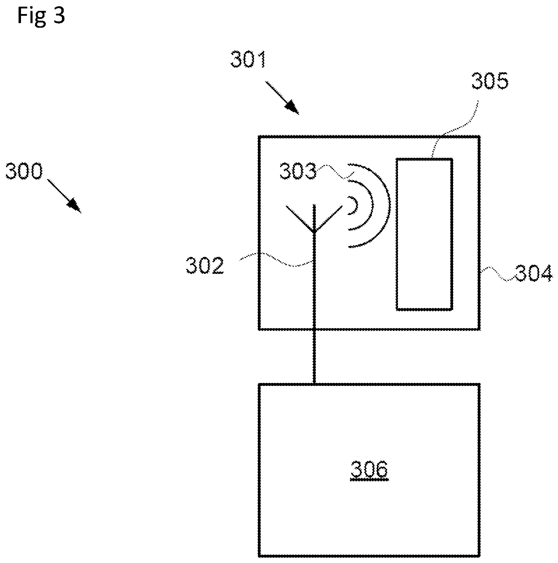

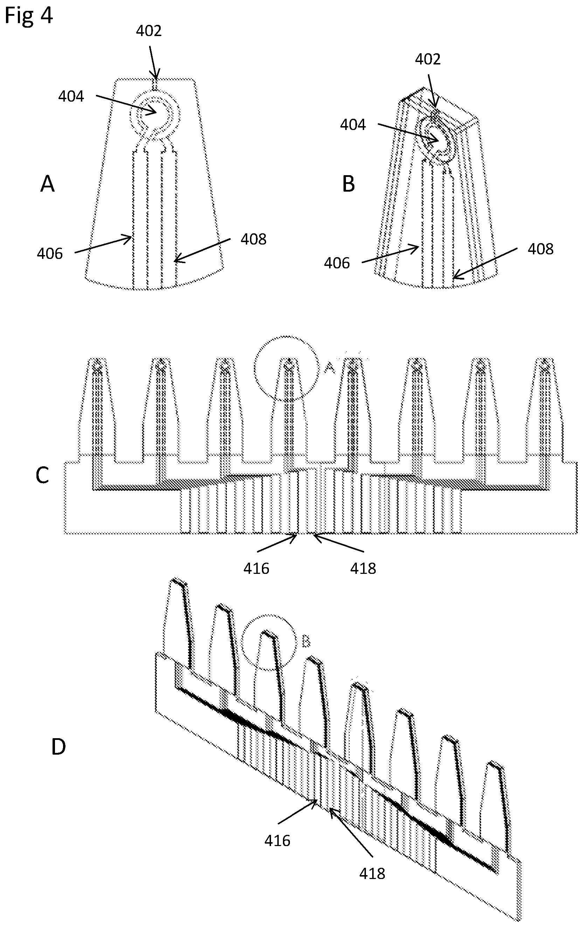

Figures 3 and 4 illustrate glucose sensors and are described below under the respective headings.

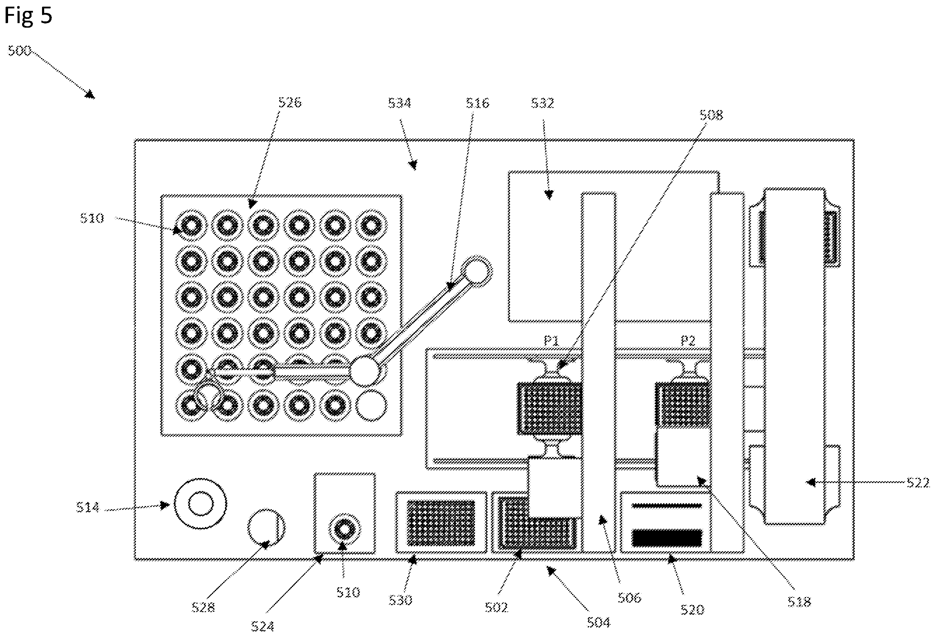

Figure 5 is an illustration of one embodiment of the system according to the invention. The system (500) comprises a plurality of microtiter plates (502), each comprising a plurality of wells constituting an array of culture medium containers, are stored in a plate storage (504). The plates loaded into the plate storage (504) preferably have predetermined amounts of antibiotic agent(s) deposited in the wells. A plate loader (506) is configured to retrieve a plate (502) from the plate storage (504) and transport it to a plate slider (508). The plate slider (508) keeps the plate in a first position (P1) for transfer of culture medium from a blood culture bottle (510) arranged in the holding rack (512) for blood culture bottles, or from a culture medium storage container (514) containing fresh culture medium. The transfer of the culture medium is effected by a robotic arm (516) equipped with a pipette head.

The plate slider (508) is adapted to move the plate (502) to a second position (P2) where the plate (502) is accessible to a measurement head (518). The measurement head (518) has mounted thereon at least one glucose sensor, preferably an array of glucose sensors, as previously described. The measurement head (518) is movable to position the glucose sensor into the wells in the plate (502) and execute a measurement of the glucose concentrations in the respective wells. The measurement head (518) may retrieve disposable glucose sensors or glucose sensor arrays in a sensor storage (520).

The plate slider is also adapted to move the plate (502) into a plate storage (522), wherein a plurality of plates can be stored between glucose measurement under conditions allowing growth of any microorganisms present in the wells.

The robotic arm (516) may also be provided with a grip for transferring blood culture bottles (510) from a blood culture bottle loading dock (524) to a holding rack (526). The holding rack (526) may, as described elsewhere, provide agitation to the blood culture bottles held in the rack. The system (500) may also be equipped with a barcode reader (528) for reading barcodes on blood culture bottles loaded into system (500) through the blood culture bottle loading dock (524). The system may also comprise a disposable pipette tip storage (530), from which the robotic arm (516) may retrieve unused pipette tips before transferring culture medium between containers. The system also preferably comprise at least one waste container (532) where used disposable units, such as pipette tips and sensor arrays, may be deposited. The fixed parts of the system are mounted on a workbench (534).

Some parts of the system, such as the computer, communication line, and housing, have been omitted in Fig. 5 to increase clarity.

### Glucose sensors

### Microwave measurements

In one embodiment, the glucose sensor functions by way of a frequency measurement of a signal indicative of the glucose concentration in the sample. The signal may be an electrical signal corresponding to a measured frequency. This is useful since a measurement method of glucose concentration uses an electrical signal to measure the permittivity of the sample, wherein the permittivity is correlated to the glucose concentration. The permittivity may be measured by a capacitive measurement method or a high frequency measurement assessing the dielectric properties of the sample.

The frequency measurement may be a measurement of a resonance frequency of the measurement chamber. This way the permittivity of the sample is easily and precisely measured.

The frequency measurement may be an electrical high frequency measurement. This electrical high frequency measurement may be a measurement of return-loss. This allows a precise measurement of the resonance frequency of the measurement chamber.

The glucose measurement apparatus may be configured to measure the glucose concentration of the sample by means of an electromagnetic field in a measurement chamber. This allows sensing the permittivity of the sample which is proportional to the glucose concentration.

The apparatus configured to measure the glucose concentration by means of an electromagnetic field may be configured to measure the electromagnetic resonance frequency of the measurement chamber. This may involve adapting the measurement chamber to a specific electromagnetic mode which propagates in a waveguide prior to entering the chamber. It is especially preferred if the measurement chamber is a micromechanical cavity since this allows for very small sample volumes and very precise measurements.

In FIG. 3 a glucose measurement module, generally denoted (300), is disclosed. The glucose measurement module (300) comprises a sensor unit (301) with an antenna (302) configured to excite an electromagnetic wave (303) in a measurement chamber (304). The measurement chamber (304) is dimensioned to be in resonance for a predetermined frequency of the electromagnetic wave. The sensor unit (301) further comprises a sample holder (305) configured to hold a sample in a position relative the measurement chamber (304). The sample holder may be a through hole for a capillary extending through the measurement chamber (304), thus providing easy insertion of a liquid culture medium sample to the measurement chamber (304).

The module further comprises a control unit (306), which comprises a tuneable oscillator and an amplifier for providing the antenna (302) with a RF signal. The control unit is further configured to measure for example the return-loss from the measurement chamber, which provides a way to determine the resonance frequency of the measurement chamber. If a sample containing glucose is placed in the sample holder, the permittivity of the glucose will change the resonance frequency of the measurement chamber. By detecting the change of resonance frequency an indication of a changed glucose concentration in the sample is provided. It is important to notice that the control unit (306) is mainly configured to detect a frequency difference; this means that no calibration of resonance frequency to glucose concentration is necessary as long as the measurements are performed with the same control unit.

A culture medium sample may be drawn into a capillary tube from a culture medium container and transferred to the sample holder (305) by a liquid handling robot for measurement of the glucose concentration. After measurement, the culture medium may be returned to the culture medium container or discarded. The capillary tube is then either discarded or transported to a washing module configured to thoroughly rinse the capillary tube of old culture medium.

### Enzyme electrodes

In one embodiment, the glucose sensor comprises an enzyme electrode. Enzyme electrodes generally comprise the enzyme glucose oxidase immobilized on a solid substrate. One such enzyme electrode was described already in 1984 (Cass et al., Anal. Chem. 1984, 56, 667-671).The electrode uses a substituted ferricinium ion as a mediator of electron transfer between immobilized glucose oxidase and a graphite electrode. A linear current response, proportional to a glucose concentration over 1-30 mM was observed.

Enzyme electrodes are commonly used to measure blood glucose levels, i.a. in monitoring of diabetes patients. Enzyme electrodes are usually used together with a read-out device adapted to transform the electrical current generated by the enzyme electrode into a glucose concentration value. One such device is the WaveSense Jazz glucometer commercially available from AgaMatrix (Salem, USA).

Enzyme electrodes configured for continuous measurement of glucose concentrations are also known in the art.

In Figure 4A and 4B, an enzyme electrode sensor is illustrated. The sensor comprises a fluid inlet port (402) facilitating culture medium transport to a solid substrate (404) on which glucose oxidase is immobilized, a first electrode (406) and a second electrode (408). When glucose is oxidized by the glucose oxidase a current response can be measured between the first (406) and second (408) electrodes.

In figures 4C and 4D, an array of glucose sensors is illustrated. The array comprises eight sensors as depicted in Figure 4A and 4B. The first and second electrodes of each sensor are in electrical contact with contact elements (416, 418) configured to be in contact with a read-out device configured to transform the measured currents into glucose concentration values. These glucose concentration values can then be retrieved by the computer (108) as shown in Figures 1 and 2, and used in the calculations of susceptibility scores according to the invention.

### Further technologies

It has also been suggested to use infrared spectroscopic methods such as attenuated total reflectance coupled with wavelet transformation (ATR-WT-IR) as a precise measurement of aqueous glucose concentrations (Al-Gharabli, Sensors (Basel). 2009; 9(8): 6254-6260).

### The computer

The system further comprises a computer computer being programmed to calculate a susceptibility score reflecting the susceptibility of the glucose metabolizing microorganism to an antibiotic activity of the antibiotic agent based on a relation between the predetermined concentration of the antibiotic agent and the time-resolved glucose concentration data. This computer may be a standard, general purpose, computer. It may also be integrated or embedded in the system according to the invention.

The computer further preferably comprise conventional ouput means to provide the calculated susceptibility score to a user of the system. Output means include i.a. displays, wired or wireless communication to other units such as mobile phones, computers, tablets etc., and printers.

### Calculation of the susceptibility score

The computer is programmed to calculate a susceptibility score reflecting the susceptibility of the glucose metabolizing microorganism to an antibiotic activity of the antibiotic agent. One embodiment of this calculation is exemplified below.

A susceptibility score can be qualitative, semi-quantitative, or quantitative.

In one embodiment, the susceptibility score is a qualitative susceptibility score classifying the microorganism as "susceptible", or "non-susceptible" to an antibiotic agent at a specified concentration.

This could be calculated by comparing the rate of change in glucose concentrations over time in the reference culture medium and the culture medium containing antibiotic agent at the specified concentration, respectively. If the glucose concentration decreases more rapidly in the reference culture medium as compared to the culture medium containing antibiotic agent, then the microorganism is susceptible to the antibiotic agent and the susceptibility score is set to "susceptible". If, on the other hand, the glucose concentration decreases at the same or substantially the same rate in the reference culture medium as in the culture medium containing antibiotic agent, then the microorganism is not susceptible to the antibiotic agent and the susceptibility score is set to "non-susceptible".

In one embodiment, the assignment of the score "susceptible" is conditioned on the rate of change in glucose concentration at the specified concentration of antibiotic agent being at, or below, a threshold fraction of the rate of change of glucose concentration in the reference medium. That is, the rate of change in the presence of the antibiotic agent should no more than for example half the rate of change in the absence of the antibiotic. The exact value of the threshold fraction of rate of change may be set by the skilled person in implementing the method for various purposes, and examples include 10%, 25%, 50%, 75%.

In one embodiment, the assignment of the score "non-susceptible" is made if the microorganism cannot be assigned the score "susceptible" at the relevant antibiotic concentration.

In one embodiment, the microorganism is cultivated in a plurality of containers together containing a serial dilution of an antibiotic agent, as well as in the absence of the antibiotic agent. Typically such a serial dilution is comprised of eight twofold dilutions expected to cover at least one concentration where the microorganism is susceptible to the antibiotic activity of the agent, e.g. 8.0, 4.0, 2.0, 1.0, 0.5, 0.25, 0.125, and 0.0625 µg/mL as commonly used for vancomycin for use against *E.coli.*

The susceptibility score could then be set to "susceptible" for all concentrations of antibiotic where the rate of change is below the threshold for designating the microorganism as "susceptible", and the susceptibility score is set to "non-susceptible" for the other concentrations.

In one embodiment, the susceptibility score is a qualitative susceptibility score classifying the microorganism as "susceptible", "intermediate" or "resistant" to an antibiotic agent at a specified concentration. The microorganism may then be designated as "resistant" if the rate of change in glucose concentration is substantially the same when cultivated with and without antibiotic agent, or if the rate of change in glucose concentration in the presence of antibiotic agent is above a predetermined threshold fraction of the rate of change in glucose concentration in the reference medium, e.g. above 90% of the rate of change in glucose concentration in the reference medium.

If the rate of change in glucose concentration is below an absolute threshold, i.e. the microorganism does not consume any substantial amounts of glucose or consume glucose at a low rate, then the microorganism is classified as "susceptible" to the antibiotic agent at that concentration.

The microorganism may be designated as "intermediate" if it cannot be classified as "resistant" or "susceptible".

A susceptibility score may also be quantitative.

In one embodiment, the susceptibility score is defined as the lowest concentration of antibiotic agent at which the growth is inhibited (Minimum Inhibitory Concentration, MIC) or the lowest concentration of antibiotic agent at which the microorganism is killed (Minimum Lethal Concentration, MLC).

In order to establish a MIC value, the microorganism is cultivated in a plurality of containers together containing a serial dilution of an antibiotic agent, as well as in the absence of the antibiotic agent, as generally described above. A "blank" container comprising culture medium, but no microorganism and no antibiotic is preferably also provided.

The MIC is the lowest concentration of antibiotic agent that results in a rate of change in glucose concentration that is significantly lower than in the reference culture medium. In various embodiments, the MIC is defined as the lowest concentration of antibiotic agent that results in a rate of change in glucose concentration that is equal to or less than a specified threshold value. The exact threshold value is likely to depend on a number of factors specific to the implementation of the method. The method can be calibrated and a suitable threshold value set by calibration against reference strains from e.g. American Type Culture Collection and comparison with reference MIC values, such as those published by the European Committee on Antimicrobial Susceptibility Testing, EUCAST (www.eucast.org). Purely by means of illustration, exemplary threshold values are 90%, 85%, 80%, 75%, 70%, 65%, 60%, 55%, 50%, 45%, 40%, 35%, or 30% of the rate of change in glucose concentration in the corresponding reference culture medium.

Normally, all concentrations of antibiotic agent that are higher than the MIC identified as above result in lower rates of decrease of change in glucose concentration than the MIC. If, on the contrary, several concentrations of antibiotic agent that are higher than the MIC lead to a higher decrease of the rate of change in glucose concentration, then it can be deduced that the antibiotic activity of the agent does not in itself impair the growth of the microorganism. The MIC value can then be disregarded.

A Minimum Lethal Concentration (MLC) can be defined as the lowest concentration of antibiotic agent that results in a rate of change in glucose concentration that is indicative of killing of the microorganism, i.e. the rate of change in glucose concentration is essentially zero or is less than a threshold fraction of the rate of change of glucose concentration in the reference culture medium, such as less than 25%, less than 20%, less than 15%, less than 10%, or less than 5% of the rate of change of glucose concentration in the reference culture medium.

Reference strains of various microorganisms with known susceptibilities to antibiotic agents are available from depositary institutions such as the American Type Culture Collection (ATCC). Such reference strains may be used when setting up a method according to the present invention in order to establish the correlations between rates of change of glucose concentrations and susceptibility scores as discussed in the current specification. The European Committee on Antimicrobial Susceptibility Testing (EUCAST) also publishes quality control tables comprising reference MIC values that can be used to calibrate the method.

The test procedure is terminated when at least one of the below conditions apply:
1. A susceptibility score as defined above can be established.
2. A time deemed to limit the test has passed.
3. No decrese in glucose concentration can be detected between one or more measurement occasions.

### Statistical significance

The present invention relies in part on measurements of parameters and comparisons of measured parameters, most notably measurement and comparisons of glucose concentrations. It is noted that all measurements come with a margin of error that depends on a number of factors, including limitations in the measurement methods.

When a comparison of parameter values is made in a method according to the invention, it is preferred to determine if any difference between the parameter values is statistically significant. If the difference between the values is not statistically significant, the values are considered as being equal for the purposes of the invention. Only if the difference is statistically significant are the values considered different. This applies *mutatis mutandis* to the situation where a comparison is made to assess whether a parameter value differs to a certain degree, such as a given percentage, from a comparator parameter value.

A difference is considered as statistically significant if the probability that the measurement method provides the obtained differing parameter values despite the true parameter values being equal is below a certain threshold. The threshold is usually set at 5% (p<0.05) but may be set lower, such as at 1%, 0.5%, 0.01%, 0.005% or even lower.

Methods for determining if a difference is statistically significant are well-known in the art of statistics. Algorithms and pre-defined functions for making the determination are included in all major software tools commonly used for managing scientific measurement data, such as MS Excel (Microsoft, USA). One applicable statistical test is the *t*-test.

### Microorganisms

The system according to the invention may be used to analyse susceptibility of any glucosemetabolizing microorganism. Preferably, the microorganisms are human or animal pathogens, i.e. able to cause disease in an infected human or animal. The microorganisms may be bacteria, fungi, or protozoa.

Exemplary species of microorganisms are provided below.

Bacteria: *Actinomyces israelii, Bacillus anthracis, Bacteroides fragilis, Bordetella pertussis, Borrelia burgdorferi, Borrelia garinii, Borrelia afzelii, Brucella abortus, Brucella canis, Brucella melitensis, Brucella suis, Campylobacter jejuni, Chlamydia pneumoniae, Chlamydia trachomatis, Chlamydophila psittaci, Clostridium botulinum, Clostridium difficile, Clostridium perifringens, Clostridium tetani, Corynebacterium diphtheriae, Ehrlichia canis, Ehrlichia chaffeensis, Enterococcus faecalis, Enterococcus faecium, Escherichia coli, Francisella tularensis, Haemophilus influenzae, Helicobacter pylori, Klebsiella pneumoniae, Legionella pneumophila, Leptospira spp., Listeria monocytogenes, Mycobacterium leprae, Mycobacterium tuberculosis, Mycoplasma pneumoniae, Neisseria gonorrhoeae, Neisseria menigitidis, Pseudomonas aeruginosa, Nocardia asteroides, Rickettsia rickettsii, Salmonella typhi, Salmonella typhimurium, Salmonella spp. Shigella sonnei, Shigella dysenteriae, Staphylococcus aureus, Staphylococcus epidermidis, Staphylococcus saprophyticus, Streptococcus agalactieae, Streptococcus pneumoniae, Streptococcus pyogenes, Streptococcus viridans, Treponema pallidum, Vibrio cholerae, Yersinia pestis*

Fungi: *Candida albicans, Filobasidiella neoformans, Lenzites subferruginea, Penicillium ludwigii, Issatchenkia orientalis, Candida parapsilosis, Aspergillus flavus, Aspergillus fumigatus, Fusarium verticillioides, Hamigera insecticola, Aspergillus alabamensis, Scedosporium apiospermum, Scedosporium apiospermum, Fusarium solani, Trichophyton rubrum, Trichophyton interdigitale.*

Protozoa: *Entamoeba histolytica, Giardia lamblia, Cryptosporidium, Trichomonas vaginalis, Toxoplasma gondii, Plasmodium vivax, Plasmodium ovale, Plasmodium malariae and Plasmodium falciparum.*

### Methods

Methods that can be performed using the system according to the present invention are further detailed in a co-pending PCT application claiming priority from Swedish patent application 1751474-6Such methods are further explained below to illustrate potential use of the system according to the present invention.

Initially, a sample comprising, or suspected of comprising, a microorganism is provided or obtained. The sample could be any sample comprising, or suspected of comprising, a microorganism such as body fluids, including but not limited to whole blood, serum, plasma, cerebrospinal fluid, lymph, sweat, tears, faeces and urine; and environmental samples, including but not limited to soil, water and swabs. In a preferred embodiment the sample is a whole blood sample.

Depending on the origin and type of sample, it may be desirable to perform an initial cultivation in order to increase the cell count of the microorganisms in the sample or obtain a subsample of a single Colony Forming Unit (cfu). For instance, the sample could be divided into aliquots, serially diluted and plated onto Petri dishes containing suitable solid culture medium and cultivated over night to yield distinguishable colonies stemming from a single cfu, as known in the art. Samples from single colonies may then be transferred to a new culture medium using a properly sterilized loop to obtain a new culture originating from a single cfu. The sample may also be transferred to a blood culture medium for initial cultivation. Such cultivation is *per se* well-known in the art and is usually performed in so-called blood culture bottles (or "BC bottles"). Blood culture media include tryptic soy broth and thioglycollate broth, and are commercially available from a number of providers. A sample from this culture may then be used as a starting material in the method according to the invention.

When a microorganism is transferred to a new growth medium, usually a lag phase occurs when no immediate increase in cell numbers or cell mass takes place. The lag phase varies substantially in time depending on the type and state of the microorganism, and the contents of the former environment and the new culture medium. After the lag phase, the microorganism enter what is called the exponential or log phase, when the microorganisms are growing and dividing at the maximal rate possible given their potential, the nature of the medium and other culture conditions. After the exponential phase, the microorgansims enter a stationary phase when population growth ceases and the growth curve becomes horizontal. After the stationary phase, detrimental environmental changes like nutrient deprivation and accumulation of waste products lead to a death phase.

The method relies on monitoring growth of microorganisms by measuring the change in glucose concentration in the culture medium over time, in the presence or absence of an antibiotic agent. As this change is most significant during the exponential phase, the microorgansims are initially cultivated in a reference culture medium, comprising glucose and not comprising the antibiotic agent, for a period of time sufficient to observe a decrease in glucose concentration in the reference culture medium. When the decrease in glucose concentration in the reference culture medium is observed, it is considered that the culture has entered the exponential growth phase and that it is adequate to perform the other steps of the method.

The cultivation of microorgansims according to the method may be performed in any type of container that is suitable for such cultivation. It is presently preferred to perform the cultivation in liquid culture medium, and suitable containers are thus adapted to hold liquid culture medium. In order to automate the method and reduce the footprint of an automated system running the method, cultivation in microtiterplates is presently preferred. Microtiterplates for cultivation of microorganisms are commercially available from a wide range of suppliers, e.g. Greiner Bio-One (Kremsmünster, Austria).

Cultivation of microorganisms is standard practice for the person of ordinary skill in the art and described in numerous textbooks in the field, i.a. the book series "Methods in Microbiology" published by Academic Press.

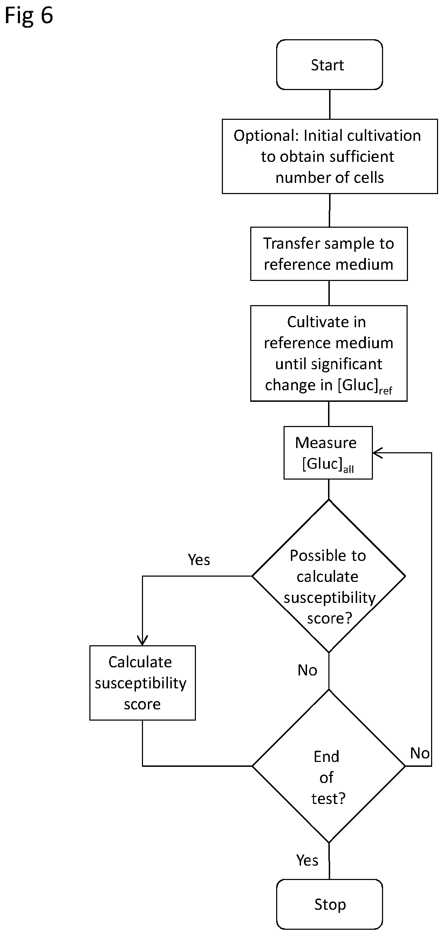

One aspect of the invention will now be described with reference to the flow diagram of FIG. 6, which illustrates a method for analysing a glucose metabolizing microorganism's susceptibility to an antibiotic agent by means of glucose measurement.

If necessary, a sample such as a blood or other body fluid sample obtained from a subject is subjected to initial cultivation in a reference culture medium (i.e. a culture medium not comprising antibiotic agent) in order to obtain a sufficient amount of viable microorganisms.

If it is considered adequate, the sample comprising microorganisms (that may or may not have been subjected to initial cultivation) is diluted with culture medium to yield a concentration of about 10⁵-10⁷ cfu/mL culture medium.

In the method, the microorganisms are then cultivated in a plurality of culture medium containers, comprising at least one reference culture medium container comprising no antibiotic agent and at least one culture medium container comprising an antibiotic agent.

A sample, or a part of a sample, is cultivated in a container comprising a culture medium comprising glucose, but no antibiotic agent. This culture medium comprising no antibiotic agent is termed reference culture medium in the present disclosure.

In one embodiment, aliquots of the sample are initially transferred to the plurality of culture medium containers before cultivation in a reference culture medium container as described. The microorganisms are thus at the same time cultivated in the culture medium containers comprising antibiotic agent and in the reference culture medium container not comprising antibiotic agent.

In another embodiment, the entire sample is transferred to a reference culture medium container for cultivation. This could correspond to the initial cultivation as described above. When a decrease in glucose concentration in this reference culture medium is observed, aliquots of this reference culture medium are transferred to the at least one reference culture medium container and at least one culture medium container comprising antibiotic agent.

The glucose concentration in the reference culture medium (denoted [Gluc]_{ref} in Fig 6) is measured continuously or at specified time intervals. The time intervals may be chosen dependent on a variety of factors, such as type of microorganism, origin of sample, type of culture medium, urgency of analysis etc., but is typically between 0.5 and 6 times per hour, such as every 10, 12, 15, 20, 30, 60 or 120 minutes.

The microorgansims are cultivated in the reference culture medium for a period of time sufficient to observe a decrease in glucose concentration in the reference culture medium. When the decrease in glucose concentration in the reference culture medium is observed, it is considered that the culture has entered, or is about to enter, the exponential growth phase and that it is adequate to perform the other steps of the method.

If aliquots of the sample have not yet been transferred to the plurality of culture medium containers, at least one aliquot of the cultivated reference culture medium is transferred to a container comprising culture medium comprising glucose and antibiotic agent at a predetermined concentration, and at least one aliquot of the cultivated reference culture medium is transferred to a container comprising reference culture medium.

The glucose concentrations in all (i.e. both the containers comprising antibiotic agent and reference medium) the culture medium containers (denoted [Gluc]ₐₗₗ in Fig 6) are then measured continuously or at specified time intervals as discussed above. A data series comprising glucose concentrations as a function of time is thus obtained for each culture medium container comprising a known concentration of antibiotic agent.

The so obtained data series can be used for calculating susceptibility scores, as further explained below. Additional glucose measurements are performed until enough data have been obtained to calculate a susceptibility score, or until one or more conditions for ending the test procedure are met. Exemplary ways to calculate susceptibility scores are further described below. Exemplary conditions for deciding whether to end the test procedure are also discussed below.

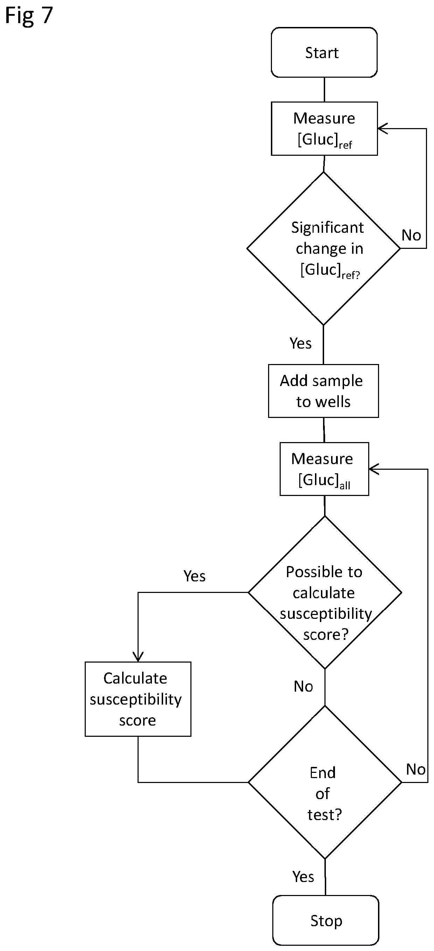

A further, more specific, embodiment of the method is disclosed in Fig 7.

In this embodiment, the method is applicable i.a. to analysis of blood samples from patients suspected of suffering from sepsis. Sepsis is a life threatening condition and mortality increases substantially with every hour that treatment is delayed. It is thus of utmost importance for a treating physician to be able to as soon as possible have information on what type of antibiotic compounds to use in treatment of the patient, and also the plasma concentrations needed to combat the infection.

In the present embodiment, a blood sample is obtained or provided and transferred to a blood culture bottle. The culture medium in the blood culture bottle then corresponds to the reference culture medium as described above, comprising glucose and no antibiotic agent.

According to standard procedures, two 10 mL samples may be obtained and transferred to one blood culture bottle for aerobic culture and one blood culture bottle for anaerobic culture. The blood culture bottles are then incubated at 37°C and gentle agitation. It is also possible to transfer a sample from the inoculated blood culture bottle to a culture medium container as used in the subsequent steps of the method and incubate the culture medium container under corresponding conditions.

The microorgansims are initially cultivated in the reference culture medium for a period of time sufficient to observe a decrease in glucose concentration in the reference culture medium. When the decrease in glucose concentration in the reference culture medium is observed, it is considered that the culture has entered, or is about to enter, the exponential growth phase and that it is adequate to perform the other steps of the method.

The glucose concentration in the reference culture medium (denoted [Gluc]_{ref} in Fig 7) is measured continuously or at specified time intervals. The time intervals may be chosen dependent on a variety of factors, such as type of microorganism, origin of sample, type of culture medium, urgency of analysis etc., but is typically between 0.5 and 6 times per hour, such as every 10, 12, 15, 20, 30, 60 or 120 minutes.

At least one aliquot of the blood culture medium is transferred to a reference culture medium container comprising no antibiotic agent.

At least one further aliquot of the sample is transferred to a container comprising antibiotic agent in a predetermined amount, yielding a predetermined concentration of the antibiotic agent in the container.

The glucose concentrations in all (i.e. both the containers comprising antibiotic agent and reference medium) the culture medium containers (denoted [Gluc]ₐₗₗ in Fig 7) are then measured continuously or at specified time intervals as discussed above. A data series comprising glucose concentrations as a function of time is thus obtained for each culture medium container comprising a known concentration of antibiotic agent.

The so obtained data series can be used for calculating susceptibility scores, as further explained below. Additional glucose measurements are performed if necessary until enough data have been obtained to calculate a susceptibility score, or until one or more conditions for ending the test procedure are met. Exemplary ways to calculate susceptibility scores are further described below. Exemplary conditions for deciding whether to end the test procedure are also discussed below.

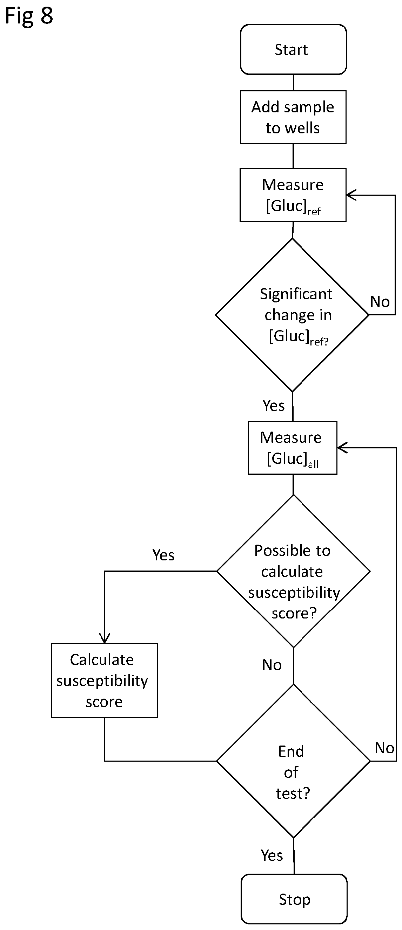

A further embodiment of the method is disclosed in Fig 8.

In this embodiment, the method is applicable i.a. to analysis of samples having a higher initial concentration of colony forming units of microorganisms, which reduces the time needed for cultivation.

In the present embodiment, aliquots of the sample are transferred to at least one container comprising reference culture medium, and at least one container comprising culture medium comprising an antibiotic agent. In a presently preferred embodiment, an aliquot is transferred to a volume of culture medium that is 99 times the volume of the aliquot, e.g. 10 µl sample is transferred to 990 µl culture medium. If the sample is from a culture on solid medium, a 1 µl loop of microorganisms may be transferred to 1 mL of liquid culture medium, and this culture medium is then diluted 1:19 to yield a suitable concentration of microorganisms.

The microorgansims are cultivated in the culture medium containers for a period of time sufficient to observe a decrease in glucose concentration in the reference culture medium. When the decrease in glucose concentration in the reference culture medium is observed, it is considered that the culture has entered, or is about to enter, the exponential growth phase and that it is adequate to perform the other steps of the method.

The glucose concentration in the reference culture medium (denoted [Gluc]_{ref} in Fig 8) is measured continuously or at specified time intervals. The time intervals may be chosen dependent on a variety of factors, such as type of microorganism, origin of sample, type of culture medium, urgency of analysis etc., but is typically between 0.5 and 6 times per hour, such as every 10, 12, 15, 20, 30, 60 or 120 minutes.

The glucose concentrations in all (i.e. both the containers comprising antibiotic agent and reference medium) the culture medium containers (denoted [Gluc]ₐₗₗ in Fig 8) are then measured continuously or at specified time intervals as discussed above. A data series comprising glucose concentrations as a function of time is thus obtained for each culture medium container comprising a known concentration of antibiotic agent.

The so obtained data series can be used for calculating susceptibility scores, as further explained below. Additional glucose measurements are performed if necessary until enough data have been obtained to calculate a susceptibility score, or until one or more conditions for ending the test procedure are met. Exemplary ways to calculate susceptibility scores are further described below. Exemplary conditions for deciding whether to end the test procedure are also discussed below.

### Experimental protocol

The following procedures illustrate in some detail one way of putting the invention into practice. The person skilled in the art will understand that various changes may be made to the protocols below without departing from the scope of the invention.

### Culture media based on Mueller Hinton Broth (MHB) for 96 well plate

Mueller Hinton broth generally comprises beef extract or beef infusion, casein hydrolysate and starch. The general proportions are 2 g/L beef extract or 300 g/L beef infusion, 17.5 g/L casein hydrolysate, and 1.5 g/L starch. The final pH is 7.3±0.2 at 25°C. (Mueller J. H. and Hinton J., 1941, Proc. Soc. Exp. Biol. Med., 48:330).

Steps that arrives at final mix in plates being 1mg/ml d-glucose and 100% MHB.

Antibiotic agents have been fixated at bottom of wells through evaporation and the amount in each well corresponds to the specified concentration as per standard when 0.1 ml of solution is added.

### Mueller Hinton broth

A. Prepare suspension
   1. Suspend 1 loop [1µl] of bacteria (cells) in 1ml MHB [100%]. Vortex until fully suspended
   2. Label the test tube and put it in the micro tube stand.
   3. Repeat from step 1 if many suspensions are needed.
B. Prepare Culture Media Base
   1. Transfer 0.0167 ml D-Glucose stock solution [300 mg/ml] to a 5 ml centrifuge tube containing 4,983 ml MHB [100%].
   2. Vortex or swirl tube for about 10- 20 s
   3. Repeat from step 1 for a second or more tube(s) of 5 ml is/are needed.
C. Mix reference blank in the well plate (no cells no ab)
   1. By pipette, transfer 0.1 ml Ascilion Media base to the designated blank on the 96 well plate.
   2. Repeat from relevant step to obtain the number of blanks that you may need
D. Mix sample solution, dispense and measure
   1. Transfer 0.950 ml Culture Medium to a 1ml eppendorf tube and label it as sample "xxx", then put it in the microbe stand.
   2. Vortex the test tube containing your cell suspension and then, by pipette, transfer 0.05 ml to the eppendorf tube labeled as sample "xxx" and and pipette up and down three times to ensure mixing
   3. From the eppendorf tube labelled sample "xxx" transfer 0.1 ml to each well in the designated row for that particular sample as well as 0.1 ml to the designated reference well containing no antibiotic
   4. Repeat the process for each sample tube.
   5. Measure all wells, starting with the references.

### Culture media based on Brain Heart Infusion (BHI) for 96 well plate

Brain Heart Infusion broth generally comprises beef heart infusion, calf brain infusion, disodium hydrogen phosphate, D(+)-glucose, and peptone. The general proportions are beef heart (infusion from 250g), 5 g/L, calf brains (infusion from 200g), 12.5 g/L, disodium hydrogen phosphate, 2.5 g/L, D(+)-glucose, 2 g/L, peptone, 10 g/L. final pH is 7.4±0.2 at 25°C. The (SigmaAldrich cat. # 53286). In the present invention, BHI is used with a lower concentration of glucose.

### Steps that arrives at final mix in wells being 1mg/ml d-glucose and 2% BHI

Antibiotic agents have been fixated at bottom of wells through evaporation and the amount in each well corresponds to the specified concentration as per standard when 0.1 ml of solution is added.
A. Prepare Culture Media components
   1. Directly in a centrifuge tube or in a glass beaker: Under stirring dissolve 1 g BHI dry powder without glucose in 25 ml of deionized water
   2. Label tube "4% BHI". If dissolved in beaker transfer solution to centrifuge tube and label it as mentioned.
   3. Prepare centrifuge tube with 10 ml of 2 mg/ml glucose solution: i.e transfer 0.067 ml of glucose stock solution [300mg/ml] to an eppendorf containing 9.933 ml of deionized water and label the tube "2 mg/ml GLU."
B. Prepare suspension
   1. Into two ependorf tube, transfer 1 ml of 4% BHI to the first and 0.5 ml of 4% BHI to the second along with 0.5 ml of deionized water.
   2. In the first, suspend 1 loop [1µl] of bacteria. Vortex until fully suspended. Label "1:1" along with name of bacteria.
   3. Transfer 0.1 ml from the 1:1 suspension above to the second eppendorf tube and pipette up and down three times. Label it "1:9 - 2%" along with name of bacteria.
   4. Repeat from step 1 if many suspensions are needed.
C. Prepare Culture Media for blanks and dispense for blank(s)
   1. Transfer 5 ml of 4% BHI to a new centrifuge tube.
   2. Then by transfer-pipette mix 5 ml from the tube labelled "2 mg/ml GLU." into the tube described above (C, step 1). Culture Media is now ready [ 2% BHI + 1mg/ml glucose]
   3. Vortex or swirl tube for about 10- 20 s and label it "Culture Medium".
   4. By pipette, transfer 0.1 ml Culture Medium to the designated blank(s) on the 96 well plate
   5. Repeat from relevant step to obtain the number of blanks that you may need
D. Mix sample solution, dispense and measure
   1. Transfer 0.5 ml of Culture Medium to an eppedorf tube and label in "1:19"
   2. After vortexing for 10s, transfer 0.5 ml of the cell suspension labelled "1:9 - 2%" to the test tube above (D, step 1) and label it additional as sample "xxx" - pipette up and down three times to ensure mixing
   3. Now from the eppendorf tube you just labelled "1:19" and sample "xxx", transfer 0.1 ml to each well in the designated row for that particular sample as well as 0.1 ml to the designated reference well containing no antibiotic
   4. Repeat the process for each sample tube.
   5. Measure all wells, starting with the references.

The present invention is not limited to the above-described preferred embodiments or the examples provided below. Various alternatives, modifications and equivalents may be used. Therefore, the above embodiments should not be taken as limiting the scope of the invention, which is defined by the appended claims.

### Examples

### Example 1: Quantitative susceptibility score

Strains of *Escherichia coli* (ATCC 25922), *Enterococcus faecalis* (ATCC 29212) and *Staphylococcus aureus* (ATCC 29213) were inoculated in blood culture bottles (SVA, Uppsala, Sweden) and cultivated according to the manufacturer's instructions until positive.

Medium from each blood culture bottle was diluted 1:100 with cation adjusted Mueller-Hinton broth (SVA, Uppsala, Sweden) supplemented with glucose to a concentration of 1.0 mg/mL.

100 µL medium from blood culture bottles was added to wells of a microtitre plate comprising serial dilutions of the antibiotic agents vancomycin, gentamicin, and cefoxitin (all obtained from Sigma Aldrich), according to Table 1.

**Table 1: Concentrations of antibiotic agents are given in µg/mL. C=Cefoxitin; G=Gentamicin; V=Vancomycin; Ref=Bacteria with no antibiotic; Blank=Only medium; RBC; Red Blood Cells in medium**

| | 1 | 2 | 3 | 4 | 5 | 6 | 7 | 8 | 9 | 10 | 11 | 12 |
|---|---|---|---|---|---|---|---|---|---|---|---|---|
| A | | | | | | C 32 | | G 32 | | V 8 | | Ref |
| B | | | | | | C 16 | | G 16 | | V 4 | | Blank |
| C | | | | | | C 8 | | G 8 | | V 2 | | RBC |
| D | | | | | | C 4 | | G 4 | | V 1 | | |
| E | | | | | | C 2 | | G 2 | | V 0.5 | | |
| F | | | | | | C 1 | | G 1 | | V 0.25 | | |
| G | | | | | | C 0.5 | | G 0.5 | | V 0.125 | | |
| H | | | | | | C 0.25 | | G 0.25 | | V 0.0625 | | |

Glucose concentrations were measured at 0, 60, 120, 180, and 240 minutes using a WaveSense Jazz glucometer (AgaMatrix, Inc., Salem, NH, USA) with accompanying test strips. The glucose measurement device had a detection limit of 20 mg glucose per mL and no values below 20 mg/mL were recorded.

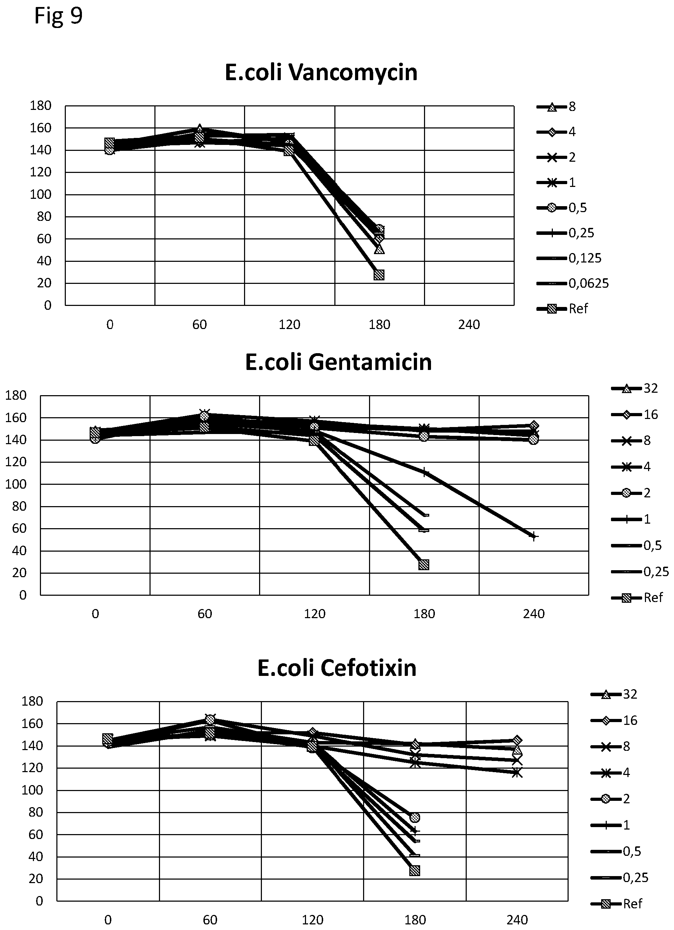

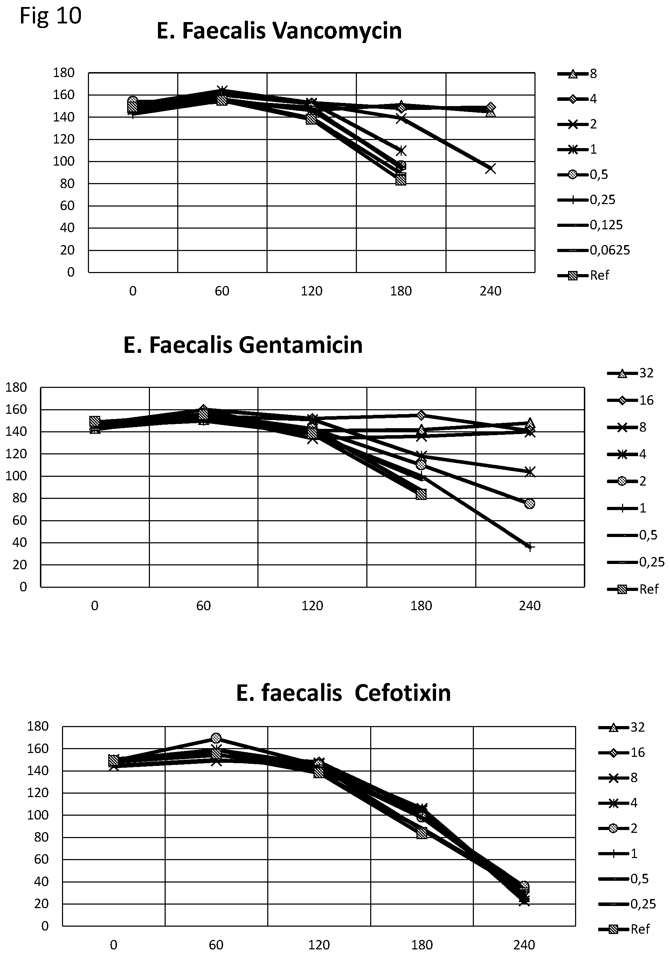

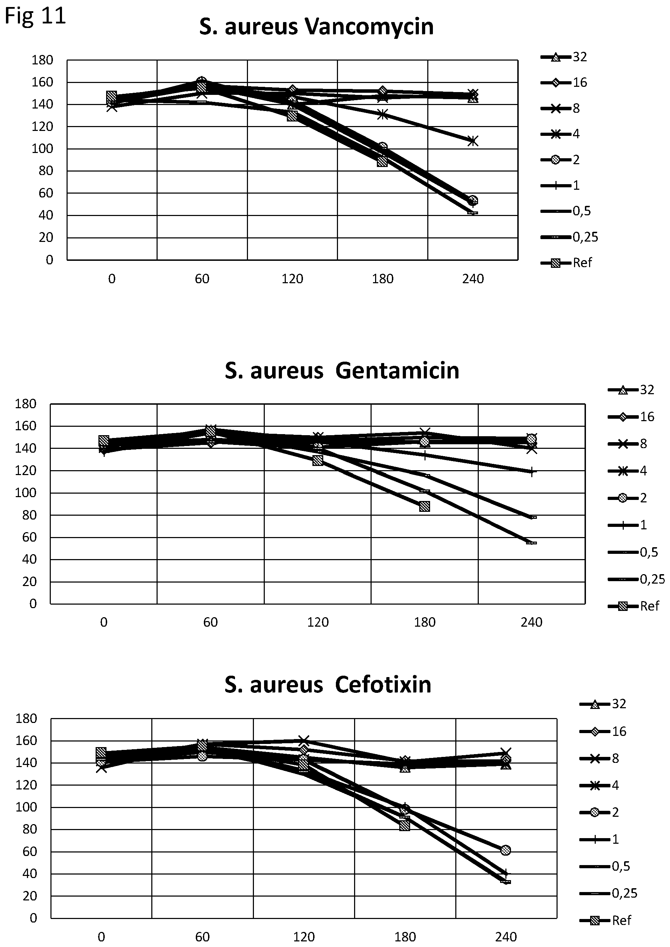

The results are shown in Tables 2, 3, 4 and figures 9, 10, and 11.

**Table 2: E.coli. C=Cefoxitin, G=Gentamicin, and V=Vancomycin. F=the rate of change in glucose concentration in relation to the reference.**

| ***Wells*** | ***[Ab]*** | ***Antibiotic*** | ***t=0*** | ***t=60*** | ***t=120*** | ***t=180*** | ***t=240*** | ***dC*/*dT*** | ***F*** |
|---|---|---|---|---|---|---|---|---|---|
| A6 | 32 | C | 146 | 150 | 143 | 142 | 137 | 3 | 5% |
| B6 | 16 | C | 142 | 152 | 152 | 141 | 145 | 4 | 6% |
| C6 | 8 | C | 145 | 164 | 149 | 132 | 127 | 11 | 18% |
| D6 | **4** | C | 146 | 149 | 140 | 125 | 116 | 12 | **19%** |
| E6 | 2 | C | 143 | 163 | 138 | 75 | | 63 | 102% |
| F6 | 1 | C | 144 | 150 | 143 | 63 | | 80 | 129% |
| G6 | 0,5 | C | 139 | 154 | 140 | 54 | | 86 | 139% |
| H6 | 0,25 | C | 145 | 157 | 143 | 41 | | 102 | 165% |
| A8 | 32 | G | 148 | 158 | 155 | 150 | 147 | 4 | 6% |
| B8 | 16 | G | 143 | 155 | 152 | 149 | 153 | 1 | 1% |
| C8 | 8 | G | 144 | 156 | 153 | 150 | 144 | 5 | 7% |
| D8 | 4 | G | 147 | 163 | 157 | 148 | 148 | 5 | 7% |
| E8 | 2 | G | 141 | 161 | 151 | 143 | 140 | 6 | 9% |
| F8 | 1 | G | 144 | 155 | 148 | 111 | 53 | 48 | **77%** |
| G8 | 0,5 | G | 144 | 147 | 147 | 72 | | 75 | 121% |
| H8 | 0,25 | G | 149 | 152 | 144 | 58 | | 86 | 139% |
| A10 | 8 | V | 145 | 159 | 147 | 51 | | 54 | 87% |
| B10 | 4 | V | 145 | 147 | 145 | 61 | | 43 | 69% |
| C10 | 2 | V | 144 | 147 | 151 | 67 | | 40 | 65% |
| D10 | 1 | V | 141 | 155 | 149 | 65 | | 45 | 73% |
| E10 | 0,5 | V | 140 | 149 | 145 | 68 | | 41 | 65% |
| F10 | 0,25 | V | 146 | 150 | 145 | 66 | | 42 | 68% |
| G10 | 0,125 | V | 148 | 153 | 154 | 65 | | 44 | 71% |
| H10 | 0,0625 | V | 143 | 148 | 144 | 65 | | 42 | 67% |
| A12 | - | no ab | 146 | 151 | 139 | 27 | | 62 | 100% |
| B12 | - | no ab | 145 | 151 | 160 | 151 | 155 | | |
| C12 | - | no ab | 149 | 160 | 168 | 154 | 162 | | |

**Table 3: E. faecalis. C=Cefoxitin, G=Gentamicin, and V=Vancomycin. F=the rate of change in glucose concentration in relation to the reference.**

| ***Wells*** | ***[Ab]*** | ***Antibiotic*** | ***t=0*** | ***t=60*** | ***t=120*** | ***t=180*** | ***t=240*** | ***dC*/*dT*** | ***F*** |
|---|---|---|---|---|---|---|---|---|---|
| A6 | 32 | C | 148 | 155 | 140 | 102 | 27 | 27 | 74% |
| B6 | 16 | C | 148 | 154 | 148 | 105 | 25 | 25 | 68% |
| C6 | 8 | C | 144 | 149 | 147 | 104 | 23 | 23 | 63% |
| D6 | 4 | C | 150 | 159 | 146 | 106 | 27 | 27 | 74% |
| E6 | 2 | C | 149 | 169 | 145 | 98 | 36 | 36 | 99% |
| F6 | 1 | C | 148 | 159 | 142 | 98 | 31 | 31 | 85% |
| G6 | 0,5 | C | 145 | 150 | 144 | 87 | 32 | 32 | 88% |
| H6 | 0,25 | C | 151 | 155 | 138 | 88 | 34 | 34 | 93% |
| | | | | | | | | | |
| A8 | 32 | G | 143 | 151 | 141 | 142 | 148 | 1 | 3% |
| B8 | 16 | G | 147 | 160 | 152 | 155 | 141 | 6 | 18% |
| C8 | 8 | G | 143 | 155 | 134 | 136 | 140 | 5 | 14% |
| D8 | 4 | G | 147 | 154 | 151 | 118 | 104 | 17 | 46% |
| E8 | 2 | G | 147 | 156 | 143 | 110 | 75 | 27 | **75%** |
| F8 | 1 | G | 145 | 150 | 138 | 100 | 36 | 38 | 106% |
| G8 | 0,5 | G | 146 | 160 | 138 | 97 | | 53 | 148% |
| H8 | 0,25 | G | 142 | 156 | 143 | 87 | | 52 | 144% |
| | | | | | | | | | |
| A10 | 8 | V | 150 | 156 | 146 | 151 | 145 | 3 | 7% |
| B10 | 4 | V | 147 | 162 | 153 | 148 | 149 | 7 | 19% |
| C10 | 2 | V | 147 | 160 | 152 | 139 | 94 | 11 | 29% |
| D10 | 1 | V | 151 | 164 | 153 | 110 | | 27 | **75%** |
| E10 | 0,5 | V | 154 | 155 | 148 | 96 | | 30 | 82% |
| F10 | 0,25 | V | 143 | 155 | 146 | 96 | | 30 | 82% |
| G10 | 0,125 | V | 148 | 154 | 149 | 94 | | 30 | 83% |
| H10 | 0,0625 | V | 145 | 156 | 139 | 89 | | 34 | 93% |
| A12 | - | no ab | 149 | 155 | 138 | 83 | | 36 | 100% |
| B12 | - | no ab | 152 | 166 | 155 | 167 | | | |
| C12 | - | no ab | 150 | 160 | 153 | 155 | | | |

**Table 4: S. aureus. C=Cefoxitin, G=Gentamicin, and V=Vancomycin. F=the rate of change in glucose concentration in relation to the reference.**

| ***Wells*** | ***[Ab]*** | ***Antibiotic*** | ***t=0*** | ***t=60*** | ***t=120*** | ***t=180*** | ***t=240*** | ***dC*/*dT*** | ***F*** |
|---|---|---|---|---|---|---|---|---|---|
| A6 | 32 | C | 145 | 154 | 145 | 136 | 139 | 9 | 27% |
| B6 | 16 | C | 140 | 157 | 152 | 142 | 142 | 8 | 22% |
| C6 | 8 | C | 136 | 157 | 160 | 141 | 149 | 8 | 24% |
| D6 | 4 | C | 142 | 150 | 143 | 139 | 139 | 6 | 16% |
| E6 | **2** | C | 141 | 146 | 143 | 98 | 61 | 24 | **72%** |
| F6 | 1 | C | 147 | 155 | 133 | 100 | 40 | 28 | 82% |
| G6 | 0,5 | C | 144 | 155 | 130 | 91 | 32 | 32 | 96% |
| H6 | 0,25 | C | 143 | 152 | 132 | 91 | 33 | 31 | 91% |
| | | | | | | | | | |
| A8 | 32 | G | 144 | 148 | 145 | 146 | 145 | 1 | 3% |
| B8 | 16 | G | 139 | 145 | 150 | 145 | 145 | 0 | 0% |
| C8 | 8 | G | 142 | 154 | 150 | 154 | 140 | 0 | 0% |
| D8 | 4 | G | 141 | 157 | 147 | 150 | 149 | 4 | 10% |
| E8 | 2 | G | 144 | 154 | 141 | 146 | 148 | 4 | 12% |
| F8 | 1 | G | 137 | 154 | 146 | 134 | 119 | 10 | 30% |
| G8 | 0,5 | G | 143 | 154 | 137 | 116 | 78 | 19 | 57% |
| H8 | **0,25** | G | 143 | 146 | 140 | 102 | 55 | 22 | **66%** |
| | | | | | | | | | |
| A10 | 32 | V | 145 | 157 | 140 | 148 | 146 | 5 | 13% |
| B10 | 16 | V | 146 | 157 | 153 | 152 | 149 | 3 | 7% |
| C10 | 8 | V | 138 | 150 | 150 | 146 | 149 | 2 | 6% |
| D10 | **4** | V | 142 | 157 | 147 | 131 | 107 | 13 | **39%** |
| E10 | 2 | V | 142 | 160 | 143 | 101 | 53 | 30 | 88% |
| F10 | 1 | V | 141 | 161 | 140 | 97 | 51 | 32 | 96% |
| G10 | 0,5 | V | 142 | 156 | 142 | 100 | 51 | 28 | 84% |
| H10 | 0,25 | V | 144 | 142 | 133 | 92 | 42 | 25 | 75% |
| A12 | - | no ab | 147 | 155 | 129 | 88 | | 34 | 100% |
| B12 | - | no ab | 149 | 148 | 151 | 153 | | | |
| C12 | - | no ab | 147 | 161 | 160 | 163 | | | |

### Calculation of susceptibility score

Susceptibility scores corresponding to MIC values were calculated as described above. A 80% threshold was applied, i.e. the lowest concentration of antibiotic that entailed a rate of change in glucose concentration that was equal to or less than 80% of the reference was selected as the Minimal Inhibitory Concentration. The rate of change was calculated between the measurements at 60 and 180 minutes. For vancomycin and *E.coli,* and cefoxitin and E.faecalis, MIC values of 0.0625 and 4 µg/mL, respectively, were initially obtained. However, as the rate of decrease in glucose concentration was higher for the the concentrations 8 and 32 µg/mL, respectively, it was found that the initial MIC values should be disregarded.

The results are disclosed in Table 5. The total time for obtaining the MIC values were 4 hours for all three microorganisms.

For comparison, the results were compared to reference MIC values published by the European Committee on Antimicrobial Susceptibility Testing (EUCAST) in "Routine and extended internal quality control for MIC determination and disk diffusion as recommended by EUCAST" Version 7.0, 2017.(available from http://www.eucast.org) for the same strains, *Escherichia coli* ATCC 25922, *Staphylococcus aureus* ATCC 29213, and *Enterococcus faecalis* ATCC 29212.

**Table 5. All MIC values are in µg/mL. N/A: The microorganism is not susceptible to the antibiotic agent and no MIC value can be calculated.**

| | Vancomycin | | Gentamicin | | Cefoxitin | |
|---|---|---|---|---|---|---|
| | Measured | EUCAST | Measured | EUCAST | Measured | EUCAST |
| *E.coli* | N/A | N/A | 1 | 0.5 (0.25-1) | 4 | 4 (2-8) |
| *E.faecalis* | 1 | 2 (1-4) | 2 | 8 (4-16) | N/A | N/A |
| *S.aureus* | 4 | 1 (0.5-2) | 0.25 | 0.25-0.5 (0.125-1) | 2 | 2 (1-4) |

The EUCAST MIC values are obtained by disk diffusion methodology using Mueller-Hinton agar, 35±1ºC, incubation time 18±2h. The ranges in parentheses were obtained (by EUCAST) from International Standards Organisation, ISO 20776-1: 2006.

The MIC values obtained by the method thus generally corresponds well with reference values published in the generally accepted quality control tables. The values could be obtained in just 4 hours, as compared to 16-20 hours using standard prior art technology.

### Example 2: Qualitative susceptibility score

If the susceptibility score is a qualitative susceptibility score classifying the microorganism as "susceptible", or "non-susceptible" to an antibiotic agent the answer could be given in as little as 1 hour.

Bacteria from plated cultures were suspended in cation adjusted Mueller-Hinton broth (SVA, Uppsala, Sweden) supplemented with glucose. Consequently, the concentration of microorgansims was typically two orders of magnitude higher as compared to Example 1. The concentrations of antibiotic agents were also increased as compared to Example 1. The tested microorganisms was one strain of *E. faecium,* two strains of *E.coli* and one *S. aureus.*

**Table 6. "--" denotes no measurement**

| **Bacteria** | **[Ab] µg/mL** | **Antibiotic** | **t=0** | **t=15** | **t=30** | **t=45** |
|---|---|---|---|---|---|---|
| *E. faecium* | 256 | Gentamicin | 79 | 47 | -- | -- |
| *E. coli 17* | 256 | Gentamicin | -- | 131 | 126 | 123 |
| *E. coli* ATCC 25922 | 256 | Gentamicin | -- | 156 | 155 | 136 |
| *S. aureus* | 256 | Gentamicin | -- | 137 | 139 | 131 |

The results in table 6 shows that *E. faecium* is non-susceptible towards Gentamicin and the two *E. coli* strains and the *S. aureus* are all "susceptible".

With slightly lower microbe concentrations e.g. directly from positive BC-bottles (^{~}10⁹ CFU/ml) the results are shown at least within 2 hours, where *E. coli* ATCC 25922 is classified as susceptible and *E. faecium* is non-susceptible. The results are shown in Table 7.

**Table 7. "--" denotes no measurement**

| ***Wells*** | **[Ab] µg/mL** | ***Antibiotic*** | **t=0** | **t=15** | **t=30** | **t=45** | **t=60** | **t=75** | **t=90** | **t=105** |
|---|---|---|---|---|---|---|---|---|---|---|
| *E. coli* ATCC 25922 | - | - | 144 | 137 | 130 | 101 | 73 | 42 | -- | -- |
| *E. faecium* | - | - | 146 | 156 | 148 | 144 | 136 | 122 | 94 | 55 |
| Blank | - | - | 141 | 152 | 154 | 154 | 156 | 153 | 158 | 160 |
| *E. faecium* | 256 | Gentamicin | 144 | -- | -- | 105 | 77 | 47 | | |
| *E. coli* ATCC 25922 | 256 | Gentamicin | 145 | -- | -- | 155 | 151 | 141 | 140 | 135 |

## Claims

1. A system for analysing susceptibility of a glucose metabolizing microorganism to an antibiotic agent, configured for holding at least a first and a second culture medium containers configured for holding
i. a volume of culture medium containing glucose;
ii. a sample containing a glucose metabolizing microorganism; and
iii. a predetermined concentration of the antibiotic agent;
and further configured to support culture of said glucose metabolizing microorganism for a period of time;
said system comprising
- a glucose sensor capable of measuring the glucose concentration in said culture medium in said first and second culture medium containers to provide time-resolved glucose concentration data during said period of time; and
- a computer configured to retrieve for each culture medium container:
i. data comprising the amount and/or concentration of the antibiotic agent; and
ii. the time-resolved glucose concentration data;
said computer being programmed to calculate a susceptibility score reflecting the susceptibility of the glucose metabolizing microorganism to an antibiotic activity of the antibiotic agent based on a relation between the predetermined concentration of the antibiotic agent and the time-resolved glucose concentration data.

2. The system according to claim 1, further comprising a rack configured for holding and optionally agitating at least one blood culture bottle.

3. The system according to claim 2, further comprising a robotic arm provided with a pipetting unit for transferring a predetermined amount of culture medium from a blood culture bottle and/or a storage container containing fresh culture medium to at least one culture medium container.

4. The system according to any one of claims 2 or 3, further comprising a housing covering at least said culture medium containers and said blood culture bottles, said housing being configured to maintain a controlled temperature within said housing.

5. The system according to claim 3, wherein said housing is provided with a loading dock for loading blood culture bottles into said system.

6. The system according to claim 5, further comprising a robotic arm provided with a grip for moving a blood culture bottle from the loading dock to the agitation plate.

7. The system according to claims 3 and 6, wherein the pipetting unit and the grip are provided on a single robotic arm.

8. The system according to any preceding claim, wherein the glucose sensor comprises an antenna configured to excite an electromagnetic wave in a measurement chamber and a sample holder configured to hold a culture medium sample in a position in the measurement chamber, optionally wherein the measurement chamber is dimensioned to be in resonance for a predetermined frequency of the electromagnetic wave and/or wherein the sample holder is a through hole for a capillary extending through the measurement chamber.

9. The system according to any one of claims 1-7, wherein the glucose sensor comprises an enzyme electrode.

10. The system according to any preceding claim, configured for holding a plurality of culture medium containers arranged in an array of columns and rows and optionally comprising a number of glucose sensors arranged in a linear array, wherein the number of glucose sensors is equal to the number of columns or rows in the array of culture medium containers.

11. The system according to any one of the preceding claims, further comprising means for shaking or agitation of the culture medium containers.

## Patentansprüche

1. System zum Analysieren einer Anfälligkeit eines Glukose-metabolisierenden Mikroorganismus gegenüber einem antibiotischen Mittel, das zum Halten mindestens eines ersten und eines zweiten Kulturmedienbehälters eingerichtet ist, die eingerichtet sind zum Halten
i. eines Volumens von Kulturmedium, das Glukose enthält;
ii. einer Probe, die einen Glukose-metabolisierenden Mikroorganismus enthält; und
iii. einer vorbestimmten Konzentration des antibiotischen Mittels;
und das ferner zum Unterstützen einer Kultur des Glukose-metabolisierenden Mikroorganismus für einen Zeitraum eingerichtet ist;
wobei das System Folgendes umfasst:
- einen Glukosesensor, der imstande ist, die Glukosekonzentration in dem Kulturmedium in dem ersten und dem zweiten Kulturmedienbehälter zu messen, um zeitaufgelöste Glukosekonzentrationsdaten während des Zeitraums bereitzustellen; und
- einen Computer, der dazu eingerichtet ist, für jeden Kulturmedienbehälter Folgendes abzufragen:
i. Daten, umfassend die Menge und/oder Konzentration des antibiotischen Mittels; und
ii. die zeitaufgelösten Glukosekonzentrationsdaten;
wobei der Computer dazu programmiert ist, eine Anfälligkeitswertung, welche die Anfälligkeit des Glukose-metabolisierenden Mikroorganismus gegenüber einer antibiotischen Aktivität des antibiotischen Mittels widerspiegelt, auf Grundlage einer Beziehung zwischen der vorbestimmten Konzentration des antibiotischen Mittels und den zeitaufgelösten Glukosekonzentrationsdaten zu berechnen.

2. System nach Anspruch 1, ferner umfassend ein Gestell, das zum Halten und gegebenenfalls Bewegen mindestens einer Blutkulturflasche eingerichtet ist.

3. System nach Anspruch 2, ferner umfassend einen Roboterarm, der mit einer Pipettiereinheit zum Überführen einer vorbestimmten Menge Kulturmedium aus einer Blutkulturflasche und/oder einem frisches Kulturmedium enthaltenden Lagerbehälter in mindestens einen Kulturmedienbehälter versehen ist.

4. System nach einem der Ansprüche 2 oder 3, ferner umfassend ein Gehäuse, das zumindest die Kulturmedienbehälter und die Blutkulturflaschen abdeckt, wobei das Gehäuse dazu eingerichtet ist, eine kontrollierte Temperatur innerhalb des Gehäuses aufrechtzuerhalten.

5. System nach Anspruch 3, wobei das Gehäuse mit einem Ladedock zum Laden von Blutkulturflaschen in das System versehen ist.

6. System nach Anspruch 5, ferner umfassend einen Roboterarm, der mit einem Greifer zum Befördern einer Blutkulturflasche von dem Ladedock zu der Schüttelplatte versehen ist.

7. System nach den Ansprüchen 3 und 6, wobei die Pipettiereinheit und der Greifer an einem einzelnen Roboterarm vorgesehen sind.

8. System nach einem der vorhergehenden Ansprüche, wobei der Glukosesensor eine Antenne, die dazu eingerichtet ist, eine elektromagnetische Welle in einer Messkammer anzuregen, und einen Probenhalter, der dazu eingerichtet ist, eine Kulturmedienprobe an einer Position in der Messkammer zu halten, umfasst, wobei die Messkammer gegebenenfalls so dimensioniert ist, dass sie sich bei einer vorbestimmten Frequenz der elektromagnetischen Welle in Resonanz befindet, und/oder wobei der Probenhalter ein Durchgangsloch für eine Kapillare ist, die sich durch die Messkammer erstreckt.

9. System nach einem der Ansprüche 1-7, wobei der Glukosesensor eine Enzymelektrode umfasst.

10. System nach einem der vorhergehenden Ansprüche, das zum Halten einer Vielzahl von Kulturmedienbehältern eingerichtet ist, die in einer Anordnung aus Spalten und Zeilen angeordnet ist, und gegebenenfalls umfassend eine Anzahl von Glukosesensoren, die in einer linearen Anordnung angeordnet ist, wobei die Anzahl von Glukosesensoren gleich der Anzahl von Spalten oder Zeilen in der Anordnung von Kulturmedienbehältern ist.

11. System nach einem der vorhergehenden Ansprüche, ferner umfassend Mittel zum Schütteln oder Bewegen der Kulturmedienbehälter.

## Revendications

1. Système pour analyser la sensibilité d'un micro-organisme métabolisant le glucose à un agent antibiotique, configuré pour contenir au moins un premier et un deuxième contenant de milieu de culture configurés pour contenir
i. un volume de milieu de culture contenant du glucose ;
ii. un échantillon contenant un micro-organisme métabolisant le glucose ; et
iii. une concentration prédéterminée de l'agent antibiotique ; et configuré en outre pour soutenir la culture dudit micro-organisme métabolisant le glucose pendant une période de temps ;
ledit système comprenant en outre :
- un capteur de glucose capable de mesurer la concentration de glucose dans ledit milieu de culture dans lesdits premier et deuxième contenants de milieu de culture pour fournir des données de concentration de glucose résolues dans le temps pendant ladite période de temps ; et
- un ordinateur configuré pour récupérer pour chaque contenant de milieu de culture :
i. des données comprenant la quantité et/ou la concentration de l'agent antibiotique ; et
ii. les données de concentration de glucose résolues dans le temps ;
ledit ordinateur étant programmé pour calculer un score de sensibilité reflétant la sensibilité du micro-organisme métabolisant le glucose à une activité antibiotique de l'agent antibiotique sur la base d'une relation entre la concentration prédéterminée de l'agent antibiotique et les données de concentration de glucose résolues dans le temps.

2. Système selon la revendication 1, comprenant en outre un portoir configuré pour contenir et éventuellement agiter au moins un flacon d'hémoculture.

3. Système selon la revendication 2, comprenant en outre un bras robotisé muni d'une unité de pipetage pour transférer une quantité prédéterminée de milieu de culture à partir d'un flacon d'hémoculture et/ou d'un contenant de stockage contenant du milieu de culture frais vers au moins un contenant de milieu de culture.

4. Système selon l'une quelconque des revendications 2 ou 3, comprenant en outre un boîtier recouvrant au moins lesdits contenants de milieu de culture et lesdits flacons d'hémoculture, ledit boîtier étant configuré pour maintenir une température régulée à l'intérieur dudit boîtier.

5. Système selon la revendication 3, dans lequel ledit boîtier est muni d'un quai de chargement pour charger des flacons d'hémoculture dans ledit système.

6. Système selon la revendication 5, comprenant en outre un bras robotisé muni d'une pince pour déplacer un flacon d'hémoculture à partir du quai de chargement vers la plaque d'agitation.

7. Système selon les revendications 3 et 6, dans lequel l'unité de pipetage et la pince sont prévues sur un seul bras robotisé.

8. Système selon l'une quelconque des revendications précédentes, dans lequel le capteur de glucose comprend une antenne configurée pour exciter une onde électromagnétique dans une chambre de mesure et un support d'échantillon configuré pour contenir un échantillon de milieu de culture dans une position dans la chambre de mesure, éventuellement dans lequel la chambre de mesure est dimensionnée pour être en résonance pour une fréquence prédéterminée de l'onde électromagnétique et/ou dans lequel le support d'échantillon est un trou traversant pour un capillaire s'étendant à travers la chambre de mesure.

9. Système selon l'une quelconque des revendications 1 à 7, dans lequel le capteur de glucose comprend une électrode enzymatique.

10. Système selon l'une quelconque des revendications précédentes, configuré pour contenir une pluralité de contenants de milieu de culture agencés en un réseau de colonnes et de rangées et comprenant éventuellement un certain nombre de capteurs de glucose agencés en un réseau linéaire, dans lequel le nombre de capteurs de glucose est égal au nombre de colonnes ou de rangées dans le réseau de contenants de milieu de culture.

11. Système selon l'une quelconque des revendications précédentes, comprenant en outre des moyens pour secouer ou agiter les contenants de milieu de culture.
